(19) Europäisches Patentamt / European Patent Office / Office européen des brevets

(11) **EP 4 220 662 B1**

(12) **EUROPEAN PATENT SPECIFICATION**

(45) Date of publication and mention of the grant of the patent:
**03.12.2025 Bulletin 2025/49**

(21) Application number: **22382085.3**

(22) Date of filing: **01.02.2022**

(51) International Patent Classification (IPC):
**G16H 50/20** $^{(2018.01)}$        **A61B 5/16** $^{(2006.01)}$
**A61B 5/00** $^{(2006.01)}$

(52) Cooperative Patent Classification (CPC):
**G16H 50/20; A61B 5/165; A61B 5/4088;
A61B 5/4806; A61B 5/7264**

(54) **SYSTEM  FOR DETECTING THE PRODROMAL DEVELOPMENT OF ALZHEIMER'S DISEASE FROM SLEEP PATTERNS**

SYSTEM  ZUR ERKENNUNG DER PRODROMALEN ENTWICKLUNG VON MORBUS ALZHEIMER AUS SCHLAFMUSTERN

SYSTÈME DE DÉTECTION DU DÉVELOPPEMENT PRODROMIQUE DE LA MALADIE D'ALZHEIMER À PARTIR DE SCHÉMAS DE SOMMEIL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.08.2023 Bulletin 2023/31**

(73) Proprietor: **Universidad Internacional de La Rioja (UNIR)
26006 Logroño (La Rioja) (ES)**

(72) Inventors:
• **CORBI BELLOT, Alberto
26006 Logroño (ES)**
• **BURGOS SOLANS, Daniel
26006 Logroño (ES)**

(74) Representative: **TRBL Intellectual Property
Plaza de Castilla 3, 7°A
28046 Madrid (ES)**

(56) References cited:
• **CORBI ALBERTO ET AL: "Connection between sleeping patterns and cognitive deterioration in women with Alzheimer's disease", SLEEP AND BREATHING - SCHLAF UND ATMUNG, DRUCKBILD, TITISEE-NEUSTADT, DE, vol. 26, no. 1, 1 April 2021 (2021-04-01), pages 361 - 371, XP037694246, ISSN: 1520-9512, [retrieved on 20210401], DOI: 10.1007/S11325-021-02327-X**
• **LAMPROS C. KOURTIS ET AL: "Connection between sleeping patterns and cognitive deterioration in women with Alzheimer's disease", NPJ DIGITAL MEDICINE, vol. 2, no. 1, 21 February 2019 (2019-02-21), pages 1 - 9, XP055626285, DOI: 10.1038/s41746-019-0084-2**

**Description**

**FIELD OF THE INVENTION**

The present invention belongs to the fields of translational and preventive medicine, with special focus on gerontology, cognitive deterioration, mental health, and sleep science. The invention also relates to the fields of computer-aided diagnosis, personalised medicine, machine learning, time series analysis and actigraphic monitoring.

**BACKGROUND OF THE INVENTION**

Alzheimer's disease (AD) is a type of mental decay affecting over fifty million people in developed countries. AD symptoms start with forgivable memory lapses and may later evolve towards dementia. The specific causes of this decline are still unknown, and although considerable level of understanding of the disease has been achieved to date, science has not been able to bring definitive treatments nor provide biomarkers of its progression.

Given the current lack of treatment for AD, the best strategy to counteract its progression is prevention. Experience tells that the disease is very likely to start with a process known as amnestic mild cognitive impairment, or MCI. This is a prodromal stage between the expected cognitive decline of normal ageing and a more serious mental deterioration (like that caused by AD). MCI is mainly characterised by casual short-term memory loss episodes that may increase in relevance/frequency. Within this phase, a person is still at nearly maximum brainpower (except for annoying bouts of forgetfulness). Nevertheless, MCI can also be understood as a clinically profitable stage in which it may be possible to foresee the eventual advent of AD. In this period, science is still on time to trigger the implementation of available therapies that foster the cognitive reserve of the patient. Currently, AD is diagnosed through a variety of methods that range from psychological interviews to the extraction of cerebrospinal fluids (CSF) by means of a painful lumbar puncture. However, these methods are usually applied too late to the patient, far beyond the typical MCI time span, wherein prevention therapies can no longer be of help.

Recent discoveries have established a connection between AD and sleep. Briefly, during the phase of deep sleep (often referred to as slow-wave sleep, or SWS, which consists of stage three of non-rapid eye movement sleep), the lymphatic system acts as a cleansing mechanism that sieves the poisonous conglomerates that are connected to AD. From a clinical perspective, the evolution of AD is also appreciable in sleep patterns. This has been confirmed by caregivers and quantitatively measured in controlled experimental settings. However, despite the sharp prodromal evidence of AD from sleep patterns, no solid diagnostic markers (derived from nightly behaviours) of the disease have been yet devised. Likewise, no automatic system (aimed at both personal and clinical use) has been proposed to this day that leverages sleep patterns to advance the development of AD-grounded dementia (i.e., the onset of a phase than transcends MCI).

A known technique for monitoring the prodromal development of AD is described, for instance, in A. Corbi and D. Burgos, "Connection between sleeping patterns and cognitive deterioration in women with Alzheimer's disease", Sleep. Breath. 2022 Mar, 26(1):361-371. This document discloses a system based on an inertial sensor adapted to be worn by a user, wherein the inertial sensor is adapted to measure an acceleration pattern during a period of time, and wherein the acceleration pattern comprises a set of data associated to the user's movements during sleep.

Other techniques for monitoring AD through mobile/wearable devices are described in Lampros C. Kourtis et al., "Digital biomarkers for Alzheimer's disease: the mobile/wearable devices opportunity", NPJ Digit. Med. 2019:2-9. This document discloses the relation between sleep patterns and, in particular, sleep disruption and fragmentation and the changes in prodromal stages of AD before cognitive decline. The document also points towards the use of sleep data and sleep deviations as an indicator of AD related changes long before more blatant cognitive symptoms manifest.

Despite the existence of the above techniques, in the present field there is need for the development of improved systems for monitoring sleep patterns, as well as for analysing said patterns for the detection of prodromal development of AD. The present invention is aimed to providing a solution to this need.

**SUMMARY OF THE INVENTION**

As described above, the invention provides an autonomous and affordable solution for the long-term monitoring of a user's cognitive status, suitable for the detection of mental decay.

The invention is configured for alerting of potential transitions from healthy or mildly impaired mental states to states associated with Alzheimer's disease, or similar strong dementia-related illnesses. This progression is estimated through the analysis of the bedtime behaviours (body motion) acquired during the night.

For this purpose, the invention comprises a high-precision, high-frequency, wearable motion tracking device and a personal digital setting configured for detecting, in advance, the prodromal transition from a healthy mental status, or an MCI one, to a dementia stage. The biological mechanisms behind this intellectual degeneration phase are very likely due to the advancement of AD or a similar mental decline process in a person's brain and nervous system. The invention comprises the use of an autonomous inertial sensor attached to the subject's body that allows continuously measuring acceleration data during each night (from bedtime to waking time). Subsequently, an ad hoc computing setup processes the data streams generated by the sensor, thereby obtaining transformed results such as, for instance, the absolute value of the continuous wavelet transform (CWT) coefficients (scalogram images) of the acceleration patterns. Finally, a calibrated assessment software tool, preferably based on a pre-trained convolutional neural network, performs a periodical classification on the output CWT bitmaps, for example in a daily manner. The result of this step returns the probability, for the person being monitored, of being in the process of transitioning from healthy or MCI stages to an AD-related one. The system regularly and discreetly reports the user of this progression and may be configured to emit specific warnings if the aforementioned probability exceeds an agreed confidence threshold. Both the inertial sensor and the processing unit can fit in the same housing or operate independently.

More specifically, a first object of the invention relates to a system for detecting the prodromal development of Alzheimer's disease from sleep patterns of a user, according to claim 1.

In a further preferred embodiment of the invention, the computing module is further configured with one or more filtering routines adapted to distil user's sleeping periods from awake periods, wherein the filtering routines comprise setting a plurality of filtering rules based on threshold values applied to the acceleration patterns. More preferably, the filtering routine comprises a time sliding-window low-pass filter with a threshold value **k,** that identifies one second (**t** = 1 s) quiet events $QS(t)$ and tags them with the formula:

$$QS(t) = 1 \quad \forall \quad k \quad \longrightarrow \quad k^2 > (f-1)^{-1} \times \sum_{i=1}^{f} (a_{x,y,z}(\mathbb{t}_i) - \overline{a_{x,y,z}(t)})^2 \, ,$$

wherein, otherwise, QS(t) = 0, and wherein **f** is the sampling frequency, $a_{x,y,z}$ is the acceleration in one of the three spatial axes (identified as x, y, and z) at a given instant of time $\mathbb{t}_i$ (fraction of the current second of time **t**) and $\overline{a}_{x,y,z}$ represents the average value for that 1-second window **(t).**

Even more preferably, the events where $QS(t) = 1$ are added up in time blocks, and wherein only the blocks having a length greater than a time threshold value are defined as deep sleep segments, $DS_i$. In a further preferred embodiment of the invention, the time threshold value is ten minutes, such that:

$$DS_i = \left[ \mathrm{len}(QS(t) = 1) > 600 \,\mathrm{s} \right] \, ,$$

wherein the brackets are an Iverson operator that returns 1 if the expression within the brackets is true, and 0 if false.

In a further preferred embodiment of the invention, the computing module is further configured to compute the absolute value of continuous wave transform from the aggregation of a plurality of $DS_i$ for a time period, and to generate a the two-dimensional (2D) scalogram image.

In a further preferred embodiment of the invention, the computing module is further configured to periodically report the progression of the probability of transitioning to a state characterised by Alzheimer's disease to the user, and to generate warning event information if said probability exceeds a threshold value.

In a further preferred embodiment of the invention, the inertial sensor comprises a triaxial piezoelectrical accelerometer, based on a plurality of microelectromechanical systems, wherein the accelerometer is adapted to measure acceleration data in a three-dimensional orthogonal reference system, with a frequency equal or above 50 Hz, and to assign event timestamps to said acceleration data.

In a further preferred embodiment of the invention, the inertial sensor is adapted to be worn in the wrist of the user, as a bracelet, armband, or smartwatch, such that the arm's movements of the user can be detected and translated into one or more acceleration patterns. Alternatively, or additionally, the inertial sensor can be adapted to be worn in the legs or feet of the user.

In a further preferred embodiment of the invention, the computing module is comprised in the inertial sensor. Alternatively, or additionally, the computing module and the inertial sensor comprise transmission means adapted for transmitting data from the inertial sensor to the computing module, wherein said transmission means are wired or wirelessly implemented.

A second object of the invention relates to a method for obtaining data suitable for the detection of a prodromal development of Alzheimer's disease from sleep patterns of a user. Advantageously, said method comprises the operation of a system according to any of the embodiments described in the present document, for performing the following steps:

- continuously measuring an acceleration pattern of the user during a period of time, by means of the inertial sensor, and generating a set of data associated to the user's movements during sleep;
- transmitting the data associated to the acceleration patterns from the inertial sensor to the computing module; and
- obtaining probability data of transitioning to a state characterised by Alzheimer's disease by comparison between the user's acceleration pattern and one or more reference datasets configured in the neural network.

## BRIEF DESCRIPTION OF DRAWINGS

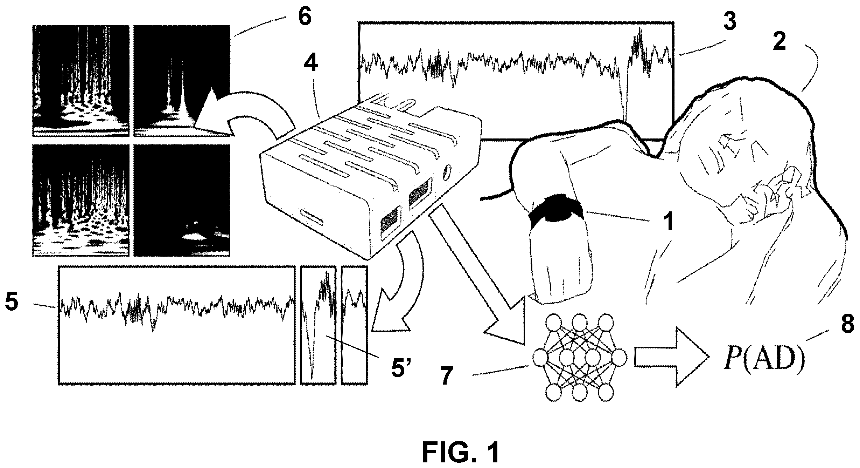

Figure 1 shows a schematic representation of the system of the invention, according to a preferred embodiment thereof.

Reference numbers used in the drawings:

(1) Inertial sensor
(2) User
(3) Acceleration pattern
(4) Computing module
(5) Sleep period
(5') Awake period
(6) Scalogram
(7) Neural network
(8) Probability of transitioning to a state characterised by Alzheimer's disease

## DETAILED DESCRIPTION OF THE INVENTION

A preferred embodiment of the invention, shown in Figure 1, will be now described for illustrative and not limiting purposes. According to this embodiment, the system of the invention comprises at least one inertial sensor (1) adapted to be worn by a user (2) whose sleep patterns are to be monitored. The inertial sensor (1) is configured to continuously measure an acceleration pattern (3) during a period of time, wherein this pattern (3) comprises a series of data associated to the user's (2) movements during sleep. For instance, the inertial sensor (1) can comprise a triaxial piezoelectrical accelerometer based on a set of microelectromechanical systems (MEMS). This instrument measures raw/analog accelerations (inertia) in a three-dimensional orthogonal reference system, with a given frequency $f$ (typically, $f \geq 50$ Hz). The generated data are later converted to units of the gravitational constant (G), through a proper analog-to-digital conversion (ADC). Also, a controller unit logs the data streams delivered by the MEMS equipment as 32-bit packed values and assigns event timestamps accordingly. In a preferred embodiment of the invention, the inertial sensor (1) can be worn in the wrist, as a bracelet, armband, or smartwatch, such that the arm's movements can be detected and translated into one or more acceleration patterns (3). However, in other embodiments of the invention the inertial sensor (1) can be worn in other parts of the user's (2) body, either alone or in combination.

The inertial sensor (1) is further configured with means for transmitting the data associated to the acceleration patterns (3) to a computing module (4), which can be comprised in the inertial sensor (1) (for example, both fitting in a same housing) or operate independently (disengaged configuration). In the latter case, the transmission of data from the inertial sensor (1) to the computing module (4) can be wired or wirelessly implemented, for example through Wi-Fi or Bluetooth communication protocols and corresponding transmission means.

The computing module (4) is preferably configured to analyse the acceleration pattern (3) datasets and to compute their associated scalograms, i.e., the absolute value of their continuous wavelet transforms, plotted as a function of time and frequency. More preferably, the computing module (4) is further configured with one or more filtering routines adapted to distil sleeping periods (5) from awake periods (5'), such that the scalogram images (6) are preferably only computed for sleeping periods. These filtering routines can be implemented by setting a plurality of filtering rules based on threshold values applied to the acceleration patterns (3), for example involving specific maximum acceleration values, or the variations thereof. In this context, the awake periods (5') typically comprise deeper acceleration value peaks, and are, in general, less stable than the sleep periods (5) (as seen in Figure 1).

For instance, in an exemplary embodiment of the invention, the filtering routine can comprise the application of a time sliding-window low-pass filter (with a threshold value $k$) that identifies one second (t = 1 s) "quiet events" QS(t) and tags them with the following formula:

$$QS(t) = 1 \quad \forall \quad k \quad \longrightarrow \quad k^2 > (f-1)^{-1} \times \sum_{i=1}^{f} (a_{x,y,z}(\mathfrak{t}_i) - \overline{a_{x,y,z}(t)})^2 \, ,$$

wherein, otherwise, QS(t) = 0, and wherein **f** is the sampling frequency, $a_{x,y,z}$ is the acceleration in one of the three spatial axes (identified as x, y, and z) at a given instant of time $\mathfrak{t}_i$ (fraction of the current second of time **t**) and $\overline{a}_{x,y,z}$ represents the average value for that 1-second window **(t)**. In a preferred embodiment of the invention, the events where $QS(t)$ = 1 are added up in time blocks. Only those blocks having a length greater than 10 minutes are defined as deep sleep segments, $DS_i$:

$$DS_i = \left[ \mathrm{len}(QS(t) = 1) > 600\,\mathrm{s} \right] \, ,$$

wherein the expression between brackets entails the Iverson formula that returns 1 if the expression within the brackets is true, and 0 if false.

Under this embodiment, the absolute value of continuous wave transform (CWT) from the aggregation of all $DS_i$ for each night is computed by the computing module (4). This delivers the two-dimensional (2D) scalogram image representing a fiduciary system in the time domain, also known as "chronofiducial". Although CWT filtering is a very widely used operation for distilling 2D images from 1D time series data, other methods can be applied in the context of the invention: Gramian Angular Fields, Markov Transition Fields, Recurrence Plot, Grey Scale Encoding, Spectrograms, etc.

Finally, the computing module (4) is configured with a neural network (7) (for example, a convolutional neural network) trained with a reference dataset of scalograms belonging to both healthy and AD patients, wherein the neural network (7) is further configured to obtain, by comparison between the user's (2) scalogram and the reference dataset, a probability (8) of transitioning to a state characterised by Alzheimer's disease is reported to the user (2).

Finally, in a preferred embodiment of the invention, the computing module (4) of the system can be further configured to regularly report the progression of the AD to the user (2), and to emit specific warnings if the aforementioned probability exceeds an agreed confidence threshold.

## Claims

1. System for detecting the prodromal development of Alzheimer's disease from sleep patterns of a user (2), said system comprising:

   - at least one inertial sensor (1) adapted to be worn by the user (2), wherein the inertial sensor (1) is further adapted to continuously measure an acceleration pattern (3) during a period of time, and wherein the acceleration pattern (3) comprises a set of data associated to the user's (2) movements during sleep;
   - a computing module (4) comprising hardware and software means;

   and wherein the inertial sensor (1) comprises means for transmitting the data associated to the acceleration patterns (3) to the computing module (4);
   wherein the software means of the computing module (4) are configured with a neural network (7) comprising a reference dataset obtained from the acceleration patterns (3) belonging to both healthy and patients of Alzheimer's disease, wherein the computing module (4) is further configured to obtain, by means of the neural network (7), a probability (8) of transitioning to a state **characterised by** Alzheimer's disease by comparison between the user's (2) acceleration pattern (3) and the reference dataset; and
   wherein the computing module (4) is further configured to generate two-dimensional information from the user's (2) acceleration pattern (3), by means of applying one of the following transformation methods to said acceleration pattern (3): continuous wave transform, Gramian angular fields, Markov transition fields, recurrence plot, grey-scale encoding, spectrograms;
   and **characterised in that** the computing module (4) is further configured to transform the acceleration pattern (3) through continuous wave transform filtering, by obtaining a scalogram (6) associated to said acceleration pattern (3).

2. System according to the preceding claim, wherein the computing module (4) is further configured with one or more filtering routines adapted to distil user's (2) sleeping periods (5) from awake periods (5'), wherein the filtering routines comprise setting a plurality of filtering rules based on threshold values applied to the acceleration patterns (3).

3. System according to the preceding claim, wherein the filtering routine comprises a time sliding-window low-pass filter with a threshold value k, that identifies one second (t = 1 s) quiet events QS(t) and tags them with the formula:

$$QS(t) = 1 \quad \forall \quad k \quad \longrightarrow \quad k^2 > (f-1)^{-1} \times \sum_{i=1}^{f} (a_{x,y,z}(t_i) - \overline{a_{x,y,z}(t)})^2 \, ,$$

wherein, otherwise, $QS(t) = 0$, and wherein **f** is the sampling frequency, $a_{x,y,z}$ is the acceleration in one of the three spatial axes (identified as x, y, and z) at a given instant of time $t_i$ (fraction of the current second of time **t**) and $\overline{a}_{x,y,z}$ represents the average value for that 1-second window **(t).**

4. System according to the preceding claim, wherein the events where $QS(t) = 1$ are added up in time blocks, and wherein only the blocks having a length greater than a time threshold value are defined as deep sleep segments, $DS_i$.

5. System according to the preceding claim, wherein the time threshold value is ten minutes, such that:

$$DS_i = \left[ \mathrm{len}(QS(t) = 1) > 600 \,\mathrm{s} \right] ,$$

wherein the brackets are an Iverson operator that returns 1 if the expression within the brackets is true, and 0 if false.

6. System according to any of claims 4-5, wherein the computing module (4) is further configured to compute the absolute value of continuous wave transform from the aggregation of a plurality of $DS_i$ for a time period, and to generate a the two-dimensional (2D) scalogram (6) image.

7. System according to any of the preceding claims, wherein the computing module (4) is further configured to periodically report the progression of the probability (8) of transitioning to a state **characterised by** Alzheimer's disease to the user (2), and to generate warning event information if said probability (8) exceeds a threshold value.

8. System according to any of the preceding claims, wherein the inertial sensor (1) comprises a triaxial piezoelectrical accelerometer, based on a plurality of microelectromechanical systems, wherein the accelerometer is adapted to measure acceleration data in a three-dimensional orthogonal reference system, with a frequency equal or above 50 Hz, and to assign event timestamps to said acceleration data.

9. System according to the preceding claim, wherein the inertial sensor (1) is adapted to be worn in the wrist of the user (2), as a bracelet, armband, or smartwatch, such that the arm's movements of the user (2) can be detected and translated into one or more acceleration patterns (3).

10. System according to any of the preceding claims, wherein the inertial sensor (1) is adapted to be worn in the legs or feet of the user (2).

11. System according to any of the preceding claims, wherein the computing module (4) is comprised in the inertial sensor (1).

12. System according to any of claims 1-10, wherein the computing module (4) and the inertial sensor (1), comprise transmission means adapted for transmitting data from the inertial sensor (1) to the computing module (4), wherein said transmission means are wired or wirelessly implemented.

13. Method for obtaining data suitable for the detection of a prodromal development of Alzheimer's disease from sleep patterns of a user (2), **characterised in that** the method comprises the operation of a system according to any of the preceding claims, for performing the following steps:

    - continuously measuring an acceleration pattern (3) of the user (2) during a period of time, by means of the inertial sensor (1), and generating a set of data associated to the user's (2) movements during sleep;
    - transmitting the data associated to the acceleration patterns (3) from the inertial sensor (1) to the computing module (4);
    - obtaining probability (8) data of transitioning to a state **characterised by** Alzheimer's disease by comparison between the user's (2) acceleration pattern (3) and one or more reference datasets configured in the neural network (7).

**Patentansprüche**

1. System zur Erkennung der prodromalen Entwicklung von Morbus Alzheimer aus Schlafmustern eines Nutzers (2), wobei das genannte System Folgendes umfasst:

   - mindestens einen Inertialsensor (1), der zum Tragen durch den Nutzer (2) vorgesehen ist, wobei der Inertialsensor (1) ferner zur kontinuierlichen Messung eines Beschleunigungsmusters (3) über einen Zeitraum hinweg ausgelegt ist, und wobei das Beschleunigungsmuster (3) einen den Schlafbewegungen des Nutzers (2) entsprechenden Datensatz umfasst;
   - ein Rechenmodul (4), das Hardware- und Softwaremittel umfasst;

   und wobei der Inertialsensor (1) Mittel zum Übermitteln der den Beschleunigungsmustern (3) entsprechenden Daten an das Rechenmodul (4) umfasst;
   wobei die Softwaremittel des Rechenmoduls (4) ein neuronales Netzwerk (7) mit einem aus den Beschleunigungsmustern (3) von gesunden Personen und Alzheimer-Patienten gewonnenen Referenzdatensatz enthalten, wobei das Rechenmodul (4) ferner dazu ausgelegt ist, mittels des neuronalen Netzwerks (7) durch Vergleich des Beschleunigungsmusters (3) des Nutzers (2) mit dem Referenzdatensatz eine Wahrscheinlichkeit (8) für den Übergang in einen durch Morbus Alzheimer gekennzeichneten Zustand zu ermitteln; und
   wobei das Rechenmodul (4) ferner zur Erzeugung zweidimensionaler Informationen aus dem Beschleunigungsmuster (3) des Nutzers (2) durch Anwendung eines der folgenden Transformationsverfahren auf das genannte Beschleunigungsmuster (3) ausgelegt ist: kontinuierliche Wavelet-Transformation, Gramian-Winkel-Felder, Markov-Übergangsfelder, Rekurrenzdiagramme, Graustufen-Codierung, Spektrogramme; und **dadurch gekennzeichnet, dass** das Rechenmodul (4) ferner dazu ausgelegt ist, das Beschleunigungsmuster (3) mittels Filterung durch kontinuierliche Wavelet-Transformation zu verarbeiten, wobei ein dem genannten Beschleunigungsmuster (3) zugeordnetes Skalogramm (6) erzeugt wird.

2. System nach vorstehendem Anspruch, wobei das Rechenmodul (4) ferner mit einer oder mehreren Filterroutinen ausgestattet ist, die dazu ausgelegt sind, die Schlafphasen (5) von den Wachphasen (5') des Nutzers (2) zu unterscheiden, wobei die Filterroutinen das Festlegen einer Vielzahl von Filterregeln auf Basis von für die Beschleunigungsmuster (3) geltenden Schwellenwerten umfassen.

3. System nach vorstehendem Anspruch, wobei die Filterroutine ein Tiefpassfilter mit gleitendem Zeitfenster mit einem Schwellenwert **k** umfasst, das einsekündige **(t** = 1 s) ruhige Ereignisse QS(t) erkennt und diese anhand der folgenden Formel kennzeichnet:

$$QS(t) = 1 \quad \forall \quad k \quad \longrightarrow \quad k^2 > (f-1)^{-1} \times \sum_{i=1}^{f} (a_{x,y,z}(\mathbb{t}_i) - \overline{a_{x,y,z}(t)})^2 \, ,$$

wobei ansonsten QS(t) = 0 ist, und wobei **f** die Abtastfrequenz darstellt, $a_{x,y,z}$ die Beschleunigung auf einer der drei Raumachsen (bezeichnet als x, y und z) zu einem bestimmten Zeitpunkt $\mathbb{t}_i$ (Bruchteil der aktuellen Sekunde **t)** ist, und $\overline{a}_{x,y,z}$ den Durchschnittswert für dieses 1-Sekunden-Fenster **(t)** angibt.

4. System nach vorstehendem Anspruch, wobei die Ereignisse, bei denen $QS(t)$ = 1, in Zeitblöcken zusammengefasst werden, und wobei nur die Blöcke, deren Länge einen Zeitschwellenwert überschreitet, als Tiefschlafsegmente $DS_i$ definiert werden.

5. System nach vorstehendem Anspruch, wobei der Zeitschwellenwert zehn Minuten beträgt, sodass

$$DS_i = [\mathrm{len}(QS(t) = 1) > 600 \, \mathrm{s}] \, ,$$

wobei die Klammern eine Prädikatabbildung darstellen, die 1 ergibt, wenn der in Klammern stehende Ausdruck wahr ist, und 0, wenn er falsch ist.

6. System nach einem der Ansprüche 4-5, wobei das Rechenmodul (4) ferner dazu ausgelegt ist, den Absolutwert der kontinuierlichen Wavelet-Transformation aus der Aggregation einer Vielzahl von $DS_i$ über einen Zeitraum zu berechnen und daraus ein zweidimensionales (2D) Skalogrammbild (6) zu erzeugen.

7. System nach einem der vorstehenden Ansprüche, wobei das Rechenmodul (4) ferner dazu ausgelegt ist, dem Nutzer (2) regelmäßig den Verlauf der Wahrscheinlichkeit (8) eines Übergangs in einen durch Morbus Alzheimer gekennzeichneten Zustand zu melden und eine Warnmeldung zu generieren, sobald die genannte Wahrscheinlichkeit (8) einen Schwellenwert überschreitet.

8. System nach einem der vorstehenden Ansprüche, wobei der Inertialsensor (1) einen auf einer Vielzahl von mikroelektromechanischen Systemen basierenden, dreiachsigen piezoelektrischen Beschleunigungsmesser umfasst, wobei der Beschleunigungsmesser dazu ausgelegt ist, Beschleunigungsdaten in einem dreidimensionalen orthogonalen Referenzsystem mit einer Frequenz von $\geq 50$ Hz zu messen und den genannten Beschleunigungsdaten Ereigniszeitstempel zuzuordnen.

9. System nach vorstehendem Anspruch, wobei der Inertialsensor (1) zum Tragen als Armreif, Armband oder Smartwatch am Handgelenk des Nutzers (2) ausgelegt ist, sodass die Armbewegungen des Nutzers (2) erfasst und in ein oder mehrere Beschleunigungsmuster (3) übersetzt werden können.

10. System nach einem der vorstehenden Ansprüche, wobei der Inertialsensor (1) zum Tragen an den Beinen oder Füßen des Nutzers (2) ausgelegt ist.

11. System nach einem der vorstehenden Ansprüche, wobei das Rechenmodul (4) in den Inertialsensor (1) integriert ist.

12. System nach einem der Ansprüche 1 bis 10, wobei das Rechenmodul (4) und der Inertialsensor (1) Übertragungsmittel umfassen, die zum Übermitteln von Daten vom Inertialsensor (1) an das Rechenmodul (4) ausgelegt sind, wobei die genannten Übertragungsmittel drahtgebunden oder drahtlos ausgeführt sind.

13. Verfahren zur Gewinnung von Daten, die zur Erkennung einer prodromalen Entwicklung von Morbus Alzheimer aus Schlafmustern eines Nutzers (2) dienen, **dadurch gekennzeichnet, dass** das Verfahren den Einsatz eines Systems nach einem der vorstehenden Ansprüche zur Durchführung der folgenden Schritte umfasst:

- kontinuierliches Messen eines Beschleunigungsmusters (3) des Nutzers (2) über einen Zeitraum hinweg mit dem Inertialsensor (1) und Erzeugen eines den Schlafbewegungen des Nutzers (2) entsprechenden Datensatzes;
- Übermitteln der den Beschleunigungsmustern (3) entsprechenden Daten vom Inertialsensor (1) an das Rechenmodul (4);
- Ermitteln von Daten hinsichtlich der Wahrscheinlichkeit (8) für den Übergang in einen durch Morbus Alzheimer gekennzeichneten Zustand durch Vergleich des Beschleunigungsmusters (3) des Nutzers (2) mit einem oder mehreren im neuronalen Netzwerk (7) hinterlegten Referenzdatensätzen.

**Revendications**

1. Système de détection du développement prodromique de la maladie d'Alzheimer à partir des schémas de sommeil d'un utilisateur (2), ledit système comprenant :

- au moins un capteur inertiel (1) adapté à être porté par l'utilisateur (2), ledit capteur inertiel (1) étant en outre adapté pour mesurer en continu un schéma d'accélération (3) pendant une période de temps, et ledit schéma d'accélération (3) comprenant un ensemble de données associées aux mouvements de l'utilisateur (2) pendant le sommeil ;
- un module de calcul (4) comprenant des moyens matériels et logiciels ;

et dans lequel le capteur inertiel (1) comprend des moyens pour transmettre les données associées aux schémas d'accélération (3) au module de calcul (4) ;

dans lequel les moyens logiciels du module de calcul (4) sont configurés avec un réseau neuronal (7) comprenant un jeu de données de référence obtenu à partir des schémas d'accélération (3) appartenant à la fois à des sujets sains et à des patients atteints de la maladie d'Alzheimer, le module de calcul (4) étant en outre configuré pour obtenir, au moyen du réseau neuronal (7), une probabilité (8) de transition vers un état **caractérisé par** la maladie d'Alzheimer par comparaison entre le schéma d'accélération (3) de l'utilisateur (2) et le jeu de données de référence ;

et dans lequel le module de calcul (4) est en outre configuré pour générer des informations bidimensionnelles à partir du schéma d'accélération (3) de l'utilisateur (2), au moyen de l'application à ledit schéma d'accélération (3) de l'une des méthodes de transformation suivantes : transformée en onde continue, champs angulaires grammiens, champs de transition de Markov, diagramme de récurrence, codage en niveaux de gris, spectrogrammes ;

et **caractérisé en ce que** le module de calcul (4) est en outre configuré pour transformer le schéma d'accélération (3) par filtrage de transformée en onde continue, en obtenant un scalogramme (6) associé audit schéma d'accélération (3).

2. Système selon la revendication précédente, dans lequel le module de calcul (4) est en outre configuré avec une ou plusieurs routines de filtrage adaptées pour extraire les périodes de sommeil (5) de l'utilisateur (2) à partir des périodes d'éveil (5'), lesdites routines de filtrage comprenant la définition d'une pluralité de règles de filtrage basées sur des valeurs seuil appliquées aux schémas d'accélération (3).

3. Système selon la revendication précédente, dans lequel la routine de filtrage comprend un filtre passe-bas à fenêtre temporelle glissante avec une valeur seuil $k$, qui identifie des événements calmes d'une seconde **(t** = 1 s) $QS(t)$ et les marque selon la formule suivante :

$$QS(t) = 1 \quad \forall \quad k \quad \longrightarrow \quad k^2 > (f-1)^{-1} \times \sum_{i=1}^{f}(a_{x,y,z}(\mathfrak{t}_i) - \overline{a_{x,y,z}(t)})^2 \, ,$$

dans lequel, autrement, $QS(t) = 0$, et dans lequel **f** est la fréquence d'échantillonnage, $a_{x,y,z}$ est l'accélération dans l'un des trois axes spatiaux (identifiés comme x, y et z) à un instant donné de temps $\mathfrak{t}_i$ (fraction de la seconde courante **t),** et $\overline{a}_{x,y,z}$ représente la valeur moyenne pour ladite fenêtre d'une seconde **(t).**

4. Système selon la revendication précédente, dans lequel les événements pour lesquels $QS(t) = 1$ sont additionnés par blocs temporels, et dans lequel seuls les blocs ayant une durée supérieure à une valeur seuil de temps sont définis comme des segments de sommeil profond, $DS_i$.

5. Système selon la revendication précédente, dans lequel la valeur seuil de temps est de dix minutes, de sorte que :

$$DS_i = [\text{len}(QS(t) = 1) > 600 \, \text{s}] \, ,$$

dans lequel les crochets représentent un opérateur d'Iverson qui renvoie 1 si l'expression entre crochets est vraie et 0 si elle est fausse.

6. Système selon l'une quelconque des revendications 4 à 5, dans lequel le module de calcul (4) est en outre configuré pour calculer la valeur absolue de la transformée en onde continue à partir de l'agrégation d'une pluralité de $DS_i$ sur une période de temps, et pour générer l'image du scalogramme bidimensionnel (2D) (6).

7. Système selon l'une quelconque des revendications précédentes, dans lequel le module de calcul (4) est en outre configuré pour signaler périodiquement à l'utilisateur (2) l'évolution de la probabilité (8) de transition vers un état **caractérisé par** la maladie d'Alzheimer, et pour générer une information d'événement d'alerte si ladite probabilité (8) dépasse une valeur seuil.

8. Système selon l'une quelconque des revendications précédentes, dans lequel le capteur inertiel (1) comprend un accéléromètre piézoélectrique triaxial, basé sur une pluralité de systèmes microélectromécaniques, ledit accéléromètre étant adapté pour mesurer des données d'accélération dans un système de référence tridimensionnel orthogonal, avec une fréquence égale ou supérieure à 50 Hz, et pour attribuer des horodatages d'événements auxdites données d'accélération.

9. Système selon la revendication précédente, dans lequel le capteur inertiel (1) est adapté pour être porté au poignet de l'utilisateur (2), sous forme de bracelet, brassard ou montre connectée, de sorte que les mouvements du bras de l'utilisateur (2) puissent être détectés et traduits en un ou plusieurs schémas d'accélération (3).

10. Système selon l'une quelconque des revendications précédentes, dans lequel le capteur inertiel (1) est adapté pour être porté sur les jambes ou les pieds de l'utilisateur (2).

11. Système selon l'une quelconque des revendications précédentes, dans lequel le module de calcul (4) est compris dans le capteur inertiel (1).

12. Système selon l'une quelconque des revendications 1 à 10, dans lequel le module de calcul (4) et le capteur inertiel (1) comprennent des moyens de transmission adaptés pour transmettre des données du capteur inertiel (1) au module de calcul (4), lesdits moyens de transmission étant mis en œuvre de manière filaire ou sans fil.

13. Procédé d'obtention de données adaptées à la détection d'un développement prodromique de la maladie d'Alzheimer à partir des schémas de sommeil d'un utilisateur (2), **caractérisé en ce que** le procédé comprend la mise en œuvre d'un système selon l'une quelconque des revendications précédentes, pour effectuer les étapes suivantes :

mesurer en continu un schéma d'accélération (3) de l'utilisateur (2) pendant une période de temps, au moyen du capteur inertiel (1), et générer un ensemble de données associées aux mouvements de l'utilisateur (2) pendant le sommeil ;
transmettre les données associées aux schémas d'accélération (3) du capteur inertiel (1) au module de calcul (4) ;
obtenir des données de probabilité (8) de transition vers un état **caractérisé par** la maladie d'Alzheimer par comparaison entre le schéma d'accélération (3) de l'utilisateur (2) et un ou plusieurs jeux de données de référence configurés dans le réseau neuronal (7).

**FIG. 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. CORBI** ; **D. BURGOS**. Connection between sleeping patterns and cognitive deterioration in women with Alzheimer's disease. *Sleep. Breath.*, March 2022, vol. 26 (1), 361-371

- **LAMPROS C. KOURTIS et al.** Digital biomarkers for Alzheimer's disease: the mobile/wearable devices opportunity. *NPJ Digit. Med.*, 2019, 2-9